# EUROPÄISCHE PATENTSCHRIFT

(11) **EP 1 448 549 B1**
(45) Veröffentlichungstag und Bekanntmachung des Hinweises auf die Patenterteilung: **15.11.2006**
(21) Anmeldenummer: 02774765.8
(22) Anmeldetag: 04.11.2002
(51) Int. Cl.: C07D 401/10, A01N 43/42

(54) **DELTA1 -PYRROLINE**
DELTA1-PYRROLINES
DELTA1 -PYRROLINES

(30) Priorität: 07.11.2001 DE 10154515
(43) Veröffentlichungstag der Anmeldung: 25.08.2004
(73) Patentinhaber: Bayer CropScience AG, 40789 Monheim (DE)
(72) Erfinder: SEITZ, Thomas, 40764 Langenfeld (DE); FÜSSLEIN, Martin, 40215 Düsseldorf (DE); JANSEN, Johannes-Rudolf, 40789 Monheim (DE); KRAATZ, Udo, 51375 Leverkusen (DE); ERDELEN, Christoph, verstorben (DE); TURBERG, Andreas, 42781 Haan (DE); HANSEN, Olaf, D 42799 Leichlingen (DE)
(86) Internationale Anmeldenummer: PCT/EP2002/012259
(87) Internationale Veröffentlichungsnummer: WO 2003/040130

(56) Entgegenhaltungen:
- WO-A-02/46151
- US-B1- 6 274 613

## Beschreibung

Die vorliegende Erfindung betrifft neue Δ¹-Pyrroline, mehrere Verfahren zu ihrer Herstellung und ihre Verwendung als Schädlingsbekämpfungsmittel.

Es ist bereits bekannt, dass zahlreiche Δ¹-Pyrroline insektizide Eigenschaften besitzen (vgl. WO 00/21958, WO 99/59968, WO 99/59967 und WO 98/22438). Die Wirksamkeit dieser Stoffe ist gut, lässt aber in manchen Fällen zu wünschen übrig.

Es wurden nun neue Δ¹-Pyrroline der Formel (I) 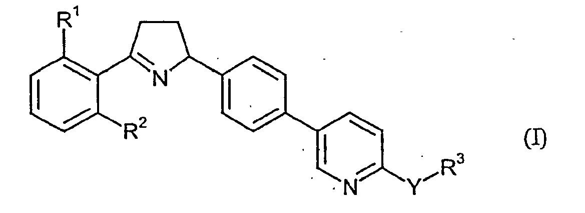 gefunden, in welcher
- R¹: für Halogen oder Methyl steht,
- R²: für Wasserstoff oder Halogen steht,
- Y: für O (Sauerstoff) oder S (Schwefel) steht,
- R³: für C₁-C₄-Alkyl, C₃-C₆-Cycloalkyl oder C₃-C₆-Cycloalkyl-C₁-C₂-alkyl steht.

Die Verbindungen der Formel (I) können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Sowohl die reinen Isomere als auch die Isomerengemische werden erfindungsgemäß beansprucht.

Weiterhin wurde gefunden, dass sich Δ¹-Pyrroline der Formel (I) herstellen lassen, indem man
A) Δ¹-Pyrroline der Formel (II) 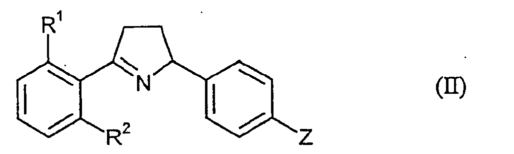 in welcher
   - R¹ und R²: die oben angegebenen Bedeutungen haben, und
   - Z: für Chlor, Brom, Iod, -OSO₂CF₃ oder -OSO₂(CF₂)₃CF₃ steht,
   mit Heterocyclen der Formel (III) 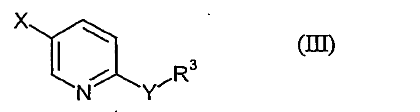 in welcher
   - Y und R³: die oben angegebenen Bedeutungen haben, und
   - X: für Chlor, Brom, Iod, -OSO₂CF₃ oder -OSO₂(CF₂)₃CF₃ steht,
   in Gegenwart eines Katalysators, in Gegenwart eines Diboronsäureesters und gegebenenfalls in Gegenwart eines Säurebindemittels und gegebenenfalls in Gegenwart eines Verdünnungsmittels in einer Tandem-Reaktion umsetzt,
   oder
B) Δ¹-Pyrroline der Formel (IV) 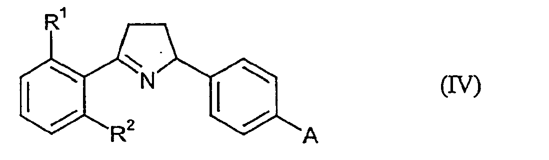 in welcher
   - R¹ und R²: die oben angegebenen Bedeutungen haben, und
   - A: für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl steht,
   mit Heterocyclen der Formel (III) 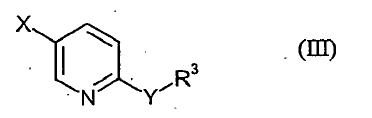 in welcher
   Y, R³ und X die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt,
   oder
C) Δ¹-Pyrroline der Formel (II) 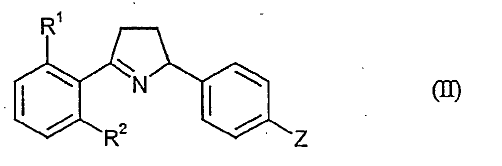 in welcher
   R¹, R² und Z die oben angegebenen Bedeutungen haben,
   mit Boronsäure-Derivaten der Formel (V) 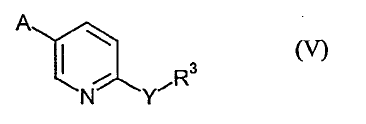 in welcher
   Y, R³ und A die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt,
   oder
D) Δ¹-Pyrroline der Formel (II-a) 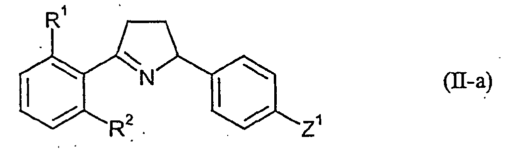
in welcher
R¹ und R² die oben angegebenen Bedeutungen haben,
Z¹ für Brom oder Iod steht,
mit organometallischen Verbindungen der Formel (VI) 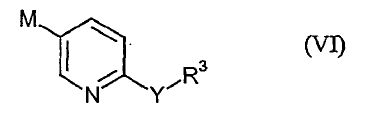 in welcher
Y und R³ die oben angegebenen Bedeutungen haben,
M für ZnCl, Sn(Me)₃ oder Sn(n-Bu)₃ steht,
in Gegenwart eines Katalysators, gegebenenfalls in Gegenwart eines Säurebindemittels und gegebenenfalls in Gegenwart eines Verdünnungsmittels umsetzt.

Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemäßen Δ¹-Pyrroline sind durch die Formel (I) allgemein definiert.

Bevorzugt sind Δ¹-Pyrroline der Formel (I), in welcher
- R¹: für Fluor, Chlor oder Methyl steht,
- R²: für Wasserstoff, Fluor oder Chlor steht,
- Y: für O (Sauerstoff) oder S (Schwefel) steht,
- R³: für C₁-C₄-Alkyl, C₃-C₆-Cycloalkyl oder C₃-C₆-Cycloalkyl-C₁-C₂-alkyl steht.

Besonders bevorzugt sind Δ¹-Pyrroline der Formel (I), in welcher
- R¹: für Fluor oder Chlor steht,
- R²: für Wasserstoff, Fluor oder Chlor steht,
- Y: für O (Sauerstoff) oder S (Schwefel) steht,
- R³: für Methyl, Ethyl, Propyl, Butyl, Cyclopropyl, Cyclobutyl, Cyclopentyl, Cyclohexyl, Cyclopropyl-C₁-C₂-alkyl, Cyclobutyl-C₁-C₂-alkyl, Cyclopentyl-C₁-C₂-alkyl oder Cyclohexyl-C₁-C₂-alkyl stehen.

Ganz besonders bevorzugt sind Δ¹-Pyrroline der Formel (I), in welcher
- R¹: für Fluor oder Chlor steht,
- R²: für Wasserstoff oder Fluor steht,
- Y: für O (Sauerstoff) oder S (Schwefel) steht,
- R³: für Methyl, Ethyl, n-Propyl, iso-Propyl, n-Butyl, iso-Butyl, sec-Butyl, tert-Butyl, Cyclopropyl, Cyclobutyl, Cyclopentyl, Cyclohexyl, Cyclopropylmethyl, Cyclobutyhmethyl, Cyclopentylmethyl, Cyclohexylmethyl, Cyclopropylethyl, Cyclobutylethyl, Cyclopentylethyl oder Cyclohexylethyl stehen.

Weiterhin bevorzugt sind Δ¹-Pyrroline der Formel (I), in welcher R¹ und R² für Fluor stehen.

Weiterhin bevorzugt sind Δ¹-Pyrroline der Formel (I), in welcher Y für Sauerstoff steht.

Weiterhin bevorzugt sind Δ¹-Pyrroline der Formel (I), in welcher Y für Schwefel steht.

Weiterhin bevorzugt sind Δ¹-Pyrroline der Formel (I), in welcher R³ für C₁-C₄-Alkyl steht.

Ganz besonders bevorzugt sind (R)-konfigurierte Verbindungen der Formel (I-a) 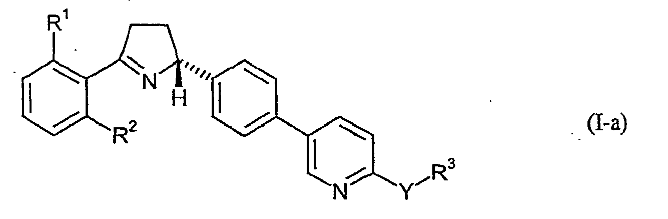 in welcher
R¹, R², Y und R³ die oben angegebenen Bedeutungen haben.

Verbindungen der Formel (I-a) erhält man durch übliche Verfahren zur Racematspaltung, wie zum Beispiel durch Chromatographie der entsprechenden Racemate an einer chiralen stationären Phase. Es ist möglich, sowohl die racemischen Endprodukte oder racemische Zwischenprodukte auf diese Weise in die beiden Enantiomere zu zerlegen.

Gesättigte Kohlenwasserstoffreste wie Alkyl können soweit möglich, jeweils geradkettig oder verzweigt sein.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Verwendet man 5-(2,6-Difluorphenyl)-2-(4-bromphenyl)-3,4-dihydro-2H-pyrrol, N-5-Brom-2-ethoxy-pyridin und 4,4,4',4',5,5,5',5'-Octamethyl-2,2'-bi-1,3,2-dioxaborolan als Ausgangsstoffe sowie einen Palladiumkatalysator, so kann der Verlauf des erfindungsgemäßen Verfahrens (A) durch das folgende Formelschema veranschaulicht werden.

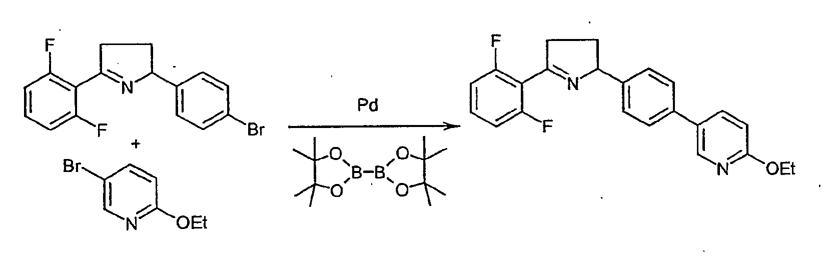

Verwendet man 5-(2,6-Difluorphenyl)-2-[4-(4,4,5,5-tetramethyl-1,3,2-dioxaborolan-2-yl)phenyl]-3,4-dihydro-2H-pyrrol und 5-Brom-2-isopropoxy-pyridin als Ausgangsstoffe sowie einen Palladiumkatalysator, so kann der Verlauf des erfindungsgemäßen Verfahrens (B) durch das folgende Formelschema veranschaulicht werden.

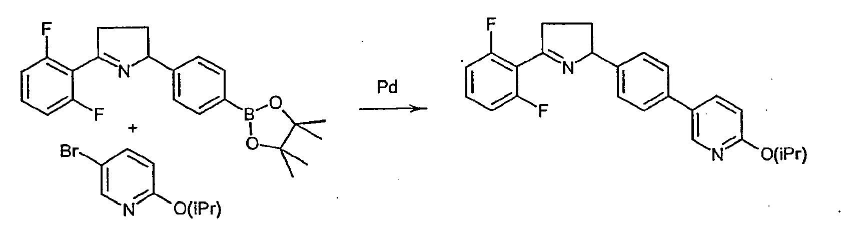

Verwendet man 5-(2,6-Difluoiphenyl)-2-[4-(trifluormethylsulfonyloxy)phenyl]-3,4-dihydro-2H-pyrrol und 2-Ethoxy-5-pyridinylboronsäure als Ausgangsstoffe sowie einen Palladiumkatalysator, so kann der Verlauf des erfindungsgemäßen Verfahrens (C) durch das folgende Formelschema veranschaulicht werden.

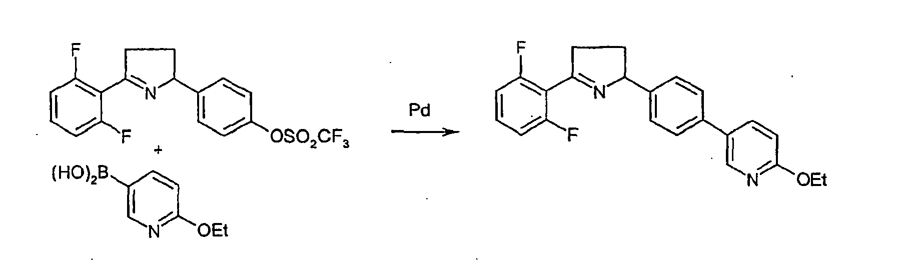

Verwendet man 5-(2,6-Difluorphenyl)-2-(4-bromphenyl)-3,4-dihydro-2H-pyrrol und 2-Ethoxy-5-(tributylstannyl)pyridin als Ausgangsstoffe sowie einen Palladiumkatalysator, so kann der Verlauf des erfindungsgemäßen Verfahrens (D) durch das folgende Formelschema veranschaulicht werden.

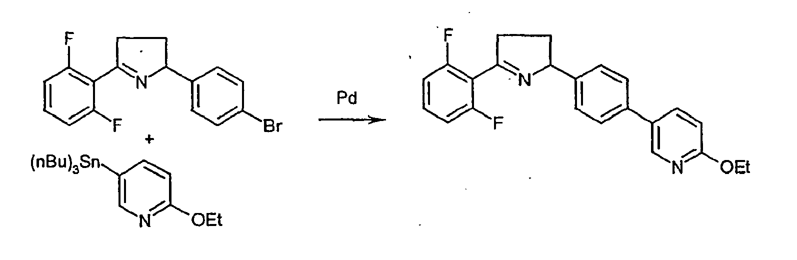

### Erläuterung der Verfahren und Zwischenprodukte

### Verfahren (A)

In einem ersten Reaktionsschritt wird eine Verbindung der Formel (II) mit einem Diboronsäureester in Gegenwart eines Palladium-Katalysators, gegebenenfalls in Gegenwart eines Säurebindemittels und gegebenenfalls in Gegenwart eines Lösungsmittels gekuppelt. Ohne Isolierung des Zwischenprodukts wird in demselben Reaktionsgefäß in einem zweiten Reaktionsschritt eine Verbindung der Formel (III) in Gegenwart eines Katalysators, gegebenenfalls in Gegenwart eines Säurebindemittels und gegebenenfalls in Gegenwart eines Lösungsmittels gekuppelt (vgl. z.B. Tetrahedron Lett. 1997, 38, 3841).

Das erfindungsgemäße Verfahren (A) kann in zwei Varianten durchgeführt werden. Es kann entweder eine Verbindung der Formel (II) oder eine Verbindung der Formel (III) vorgelegt werden. Verfahren (A) ist als Tandem-Reaktion der nachfolgend beschriebenen Verfahren (B) und (C) anzusehen.

Die bei der Durchführung des erfindungsgemäßen Verfahrens (A) als Ausgangsstoffe benötigten Δ¹-Pyrroline sind durch die Formel (II) allgemein definiert. In dieser Formel stehen R¹ und R² bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Z steht bevorzugt für Brom, Iod, -OSO₂CF₃ oder -OSO₂(CF₂)₃CF₃, besonders bevorzugt für Brom, -OSO₂CF₃ oder -OSO₂(CF₂)₃CF₃, ganz besonders bevorzugt für Brom oder -OSO₂CF₃.

Δ¹-Pyrroline der Formel (II) lassen sich nach bekannten Verfahren herstellen (vgl. WO 98/22438).

Die bei der Durchführung des erfindungsgemäßen Verfahrens (A) als Ausgangsstoffe benötigten Heterocyclen sind durch die Formel (III) allgemein definiert. In dieser Formel stehen Y und R³ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. X steht bevorzugt für Brom, Chlor, Iod oder -OSO₂CF₃, besonders bevorzugt für Brom, Chlor oder Iod, ganz besonders bevorzugt für Brom oder Chlor.

Die Heterocyclen der Formel (III) sind bekannt oder können nach bekannten Verfahren hergestellt werden (vgl. Aust. J. Chem. 1964, 17, 794; Chem. Ber. 1922, 125, 1169; Chem. Pharm. Bull. 1995, 43, 247; Eur. J. Med. Chem. 1989, 24, 249; J. Chem. Soc. C 1971, 1889; J. Chem. Soc. Perkin Trans. 1 1995, 2497; J. Med. Chem. 1991, 34, 315; J. Org. Chem. 1984, 49, 2240; J. Org. Chem. 1990, 55, 69; Org. Prep. Proced. Int. 1928, 30, 433; Synthesis 1999, 1163; Tetrahedron 1999, 40, 7975; Tetrahedron Lett. 1996, 37, 4447; Tetrahedron Lett. 2000, 41, 4335).

Als Diboronsäureester kommen bei der Durchführung des erfindungsgemäßen Verfahrens (A) 4,4,4',4',5,5,5',5'-Octamethyl-2,2'-bi-1,3,2-dioxaborolan, 5,5,5',5'-Tetramethyl-2,2'-bi-1,3,2-dioxaborinan, 4,4,4',4',6,6'-Hexamethyl-2,2'-bi-1,3,2-dioxaborinan oder 2,2'-Bi-1,3,2-benzodioxaborol in Frage. Bevorzugt verwendet man 4,4,4',4',5,5,5',5'-Octamethyl-2,2'-bi-1,3,2-dioxaborolan, 5,5,5',5'-Tetramethyl-2,2'-bi-1,3,2-dioxaborinan oder 4,4,4',4',6,6'-Hexamethyl-2,2'-bi-1,3,2-dioxaborinan, besonders bevorzugt 4,4,4',4',5,5,5',5'-Octamethyl-2,2'-bi-1,3,2-dioxaborolan oder 5,5,5',5'-Tetramethyl-2,2'-bi-1,3,2-dioxaborinan, ganz besonders bevorzugt 4,4,4',4',5,5,5',5'-Octamethyl-2,2'-bi-1,3,2-dioxaborolan.

Bei der Durchführung des erfindungsgemäßen Verfahrens (A) setzt man auf 1 Mol an Verbindung der Formel (II) im allgemeinen 1 Mol oder einen leichten Überschuss eines Diboronesters und 1 Mol oder einen leichten Überschuss einer Verbindung der Formel (III), sowie 3 % eines Palladiumkatalysators ein. Es ist jedoch auch möglich, die Reaktionskomponenten in anderen Verhältnissen einzusetzen. Es kann wahlweise die Verbindung der Formel (II) oder die Verbindung der Formel (III) zuerst vorgelegt werden. Die Aufarbeitung erfolgt nach üblichen Methoden. Im allgemeinen verfährt man in der Weise, dass man das Reaktionsgemisch mit Wasser verdünnt und mit Essigsäureethylester extrahiert. Die organische Phase wird gewaschen, getrocknet, filtriert und eingeengt. Der Rückstand wird gegebenenfalls nach üblichen Methoden, wie Chromatographie oder Umkristallisation, von eventuell noch vorhandenen Verunreinigungen befreit.

### Verfahren (B)

Die bei der Durchführung des erfindungsgemäßen Verfahrens (B) als Ausgangsstoffe benötigten △¹-Pyrroline sind durch die Formel (IV) allgemein definiert. In dieser Formel stehen R¹ und R² bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. A steht bevorzugt für (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl, besonders bevorzugt für (4,4,5,5-Tetramethyl-1,3,2-dioxaborolan)-2-yl, (5,5-Dimethyl-1,3,2-dioxaborinan)-2-yl oder (4,4,6-Trimethyl-1,3,2-dioxaborinan)-2-yl, ganz besonders bevorzugt für (4,4,5,5-Tetramethyl-1,3,2-dioxaborolan)-2-yl, (5,5-Dimethyl-1,3,2-dioxaborinan)-2-yl.

△¹-Pyrroline der Formel (IV) lassen sich herstellen, indem man
a) Verbindungen der Formel (II) 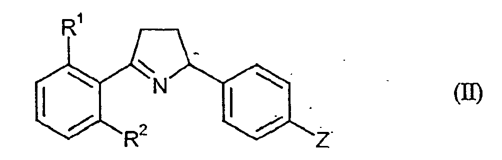 in welcher
   R¹, R² und Z die oben angegebenen Bedeutungen haben,
   mit einem Diboronsäureester in Gegenwart eines Katalysators, gegebenenfalls in Gegenwart eines Säurebindemittels und gegebenenfalls in Gegenwart eines Verdünnungsmittels umsetzt (vgl. J. Org. Chem. 1995, 60, 7508; Tetrahedron Lett. 1997, 38, 3447).

Geeignete Diboronsäureester zur Durchführung des Verfahrens (a) sind bei der Beschreibung des erfindungsgemäßen Verfahrens (A) bereits genannt worden.

Die bei der Durchführung des erfindungsgemäßen Verfahrens (B) als Ausgangsstoffe benötigten Heterocyclen der Formel (III) wurden bereits oben bei der Beschreibung des Verfahrens (A) beschrieben.
Bei der Durchführung des erfindungsgemäßen Verfahrens (B) setzt man auf 1 Mol an Verbindung der Formel (V) im allgemeinen 1 Mol oder einen leichten Überschuss an einer Verbindung der Formel (III) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (C)

Die bei der Durchführung des erfindungsgemäßen Verfahrens (C) als Ausgangsstoffe benötigten Δ¹-Pyrroline der Formel (II) wurden bereits bei der Beschreibung des Verfahrens (A) beschrieben.

Die bei der Durchführung des erfindungsgemäßen Verfahrens (C) als Ausgangsstoffe benötigten Boronsäure-Derivate sind durch die Formel (V) allgemein definiert. In dieser Formel stehen Y und R³ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. A steht bevorzugt für (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl, besonders bevorzugt für (4,4,5,5-Tetramethyl-1,3,2-dioxaborolan)-2-yl, (5,5-Dimethyl-1,3,2-dioxaborinan)-2-yl oder (4,4,6-Trimethyl-1,3,2-dioxaborinan)-2-yl, ganz besonders bevorzugt für (4,4,5,5-Tetramethyl-1,3,2-dioxaborolan)-2-yl, (5,5-Dimethyl-1,3,2-dioxaborinan)-2-yl.

Die Verbindungen der Formel (V) sind bekannt oder lassen sich nach bekannten Verfahren herstellen (vgl. J. Org. Chem. 1995, 60, 7508, Tetrahedron Lett. 1997, 38, 3447).

Bei der Durchführung des erfindungsgemäßen Verfahrens (C) setzt man auf 1 Mol an Verbindung der Formel (II) im allgemeinen 1 Mol oder einen leichten Überschuss einer Verbindung der Formel (V) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (D)

Die bei der Durchführung des erfindungsgemäßen Verfahrens (D) als Ausgangsstoffe benötigten Δ¹-Pyrroline sind durch die Formel (II-a) allgemein definiert. In dieser Formel stehen R¹ und R² bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Z¹ steht bevorzugt für Brom oder Iod.

Δ¹-Pyrroline der Formel (II-a) lassen sich nach bekannten Verfahren herstellen (vgl. WO 98/22438).

Die bei der Durchführung des erfindungsgemäßen Verfahrens (D) als Ausgangsstoffe benötigten organometallischen Verbindungen sind durch die Formel (VI) allgemein definiert. In dieser Formel stehen Y und R³ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. M steht bevorzugt für ZnCl, Sn(Me)₃ oder Sn(n-Bu)₃.

Organometallische Verbindungen der Formel (VI) sind teilweise bekannt oder können nach bekannten Methoden hergestellt werden. Es ist z.B. möglich, Verbindungen der Formel (VI) aus den entsprechenden Verbindungen der Formel (III), in welchen X für -OSO₂CF₃ steht, in situ herzustellen (vgl. Tetrahedron Lett. 1995, 36, 9085).

Bei der Durchführung des erfindungsgemäßen Verfahrens (D) setzt man auf 1 Mol an Verbindung der Formel (II-a) im allgemeinen 1 Mol oder einen leichten Überschuss einer Verbindung der Formel (VI) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Chirale Verbindungen der Formel (I-a)

Zur Herstellung chiraler Verbindungen der Formel (I-a) können beispielsweise Δ¹-Pyrroline der Formel (II-b) 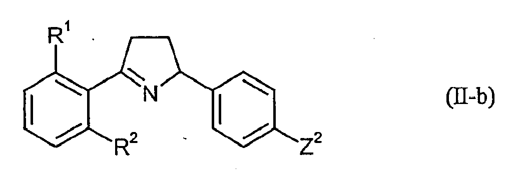 in welcher
R¹ und R² die oben angegebenen Bedeutungen haben,
Z² für Chlor, Brom oder Iod steht,
einer Racematspaltung unterzogen werden. Dabei arbeitet man beispielsweise nach Methoden der präparativen Chromatographie, vorzugsweise nach der Methode der High Performance Liquid Chromatography (HPLC). Dabei wird eine chirale stationäre Kieselgelphase verwendet. Als besonders geeignet für die Trennung der Verbindungen der Formel (II-b) in die beiden Enantiomere hat sich ein mit Tris(3,5-dimethylphenylcarbamat)-cellulose modifiziertes Kieselgel erwiesen. Dieses Trennmaterial ist kommerziell erhältlich. Es ist aber auch möglich, andere stationäre Phasen zu verwenden. Als Eluenten kommen alle üblichen inerten, organischen Solventien sowie Gemische von diesen in Frage. Vorzugsweise verwendbar sind gegebenenfalls halogenierte aliphatische, alicyclische oder aromatische Kohlenwasserstoffe, wie Petrolether, Hexan, Heptan, Cyclohexan; Dichlormethan, Chloroform; Alkohole, wie Methanol, Ethanol, Propanol; Nitrile, wie Acetonitril; Ester wie Essigsäuremethylester oder Essigsäureethylester. Besonders bevorzugt verwendet man aliphatische Kohlenwasserstoffe, wie Hexan oder Heptan, und Alkohole, wie Methanol oder Propanol, ganz besonders bevorzugt n-Heptan und Isopropanol bzw. Gemische von diesen. Im allgemeinen arbeitet man bei Temperaturen zwischen 10°C und 60°C, vorzugsweise zwischen 10°C und 40°C, besonders bevorzugt bei Raumtemperatur. Die auf diesem Wege erhaltenen (R)-konfigurierten Enantiomere werden dann als Ausgangsstoffe für die Verfahren (A), (C) oder (D) eingesetzt.

Bei der Durchführung der erfindungsgemäßen Verfahren (A), (B), (C) und (D) setzt man jeweils einen Palladium-Katalysator ein, der wiederum mit oder ohne Zusatz von weiteren Liganden verwendet werden kann. Vorzugsweise verwendet man als Katalysator PdCl₂(dppf) [dppf = 1,1'-Bis(diphenylphosphino)ferrocene], Pd(PPh₃)₄, PdCl₂(PPh₃)₂, PdCl₂(CH₃CN)₂, Pd₂(dba)₃ [dba = Dibenzylidenaceton] oder Pd(OAc)₂, besonders bevorzugt PdCl₂(dppf), Pd(PPh₃)₄, PdCl₂(PPh₃)₂, oder Pd(OAc)₂, ganz besonders bevorzugt PdCl₂(dppf) oder PdCl₂(PPh₃)₂.

Als Liganden kommen Triarylphosphine, Trialkylphosphine oder Arsine in Frage. Vorzugsweise verwendet man dppf, PPh₃, P(tert-Bu)₃, Pcy₃ oder AsPh₃, besonders bevorzugt dppf.

Als Verdünnungsmittel kommen bei der Durchführung der erfindungsgemäßen Verfahren (A), (B) und (C) jeweils alle üblichen inerten, organischen Solventien infrage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan. Besonders bevorzugt verwendet man Aceton, Dimethoxyethan, Dioxan, Tetrahydrofuran, Dimethylformamid, Dimethylacetamid, Dimethylsulfoxid, Ethanol, Toluol oder gegebenenfalls Gemische dieser genannten Verdünnungsmittel mit Wasser.

Als Verdünnungsmittel kommen bei der Durchführung des erfindungsgemäßen Verfahrens (D) jeweils alle üblichen inerten, organischen Solventien in Frage. Vorzugswiese verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol. Besonders bevorzugt verwendet man Dioxan, Tetrahydrofuran oder Toluol.

Als Säurebindemittel kommen bei der Durchführung der erfindungsgemäßen Verfahrens (A), (B), (C) und (D) jeweils alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, Alkalimetallfluoride, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert. Besonders bevorzugt verwendet man Bariumhydroxid, Natriumhydroxid, Kaliumhydroxid, Trikaliumphosphat, Caesiumcarbonat, Kaliumcarbonat, Natriumcarbonat, Kaliumacetat, Triethylamin, Kaliumtert-butanolat, Caesiumfluorid oder Kaliumfluorid.

Die Reaktionstemperaturen können bei der Durchführung der erfindungsgemäßen Verfahren (A), (B) und (C) jeweils in einem größeren Bereich variiert werden. Im allgemeinen arbeitet man bei Temperaturen zwischen 0°C und 140°C, vorzugsweise zwischen 20°C und 120°C, besonders bevorzugt zwischen 60°C und 100°C.

Die Reaktionstemperaturen können bei der Durchführung des erfindungsgemäßen Verfahrens (D) jeweils in einem größeren Bereich variiert werden. Im allgemeinen arbeitet man bei Temperaturen zwischen 0°C und 140°C, vorzugsweise zwischen 20°C und 120°C.

Bei der Durchführung aller erfindungsgemäßen Verfahren arbeitet man im allgemeinen unter Atmosphärendruck. Es ist aber auch möglich, jeweils unter erhöhtem oder vermindertem Druck zu arbeiten.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp.
Aus der Ordnung der Phthiraptera z.B. Pediculus humanus corporis, Haematopinus spp., Linognathus spp., Trichodectes spp., Damalinia spp.
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.
Aus der Ordnung der Siphonaptera z.B. Xenopsylla cheopis, Ceratophyllus spp.
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen der Formel (I) zeichnen sich insbesondere durch eine hervorragende Wirkung gegen Raupen, Käferlarven, Spinnmilben, Blattläuse und Minierfliegen aus.

Die erfindungsgemäßen Stoffe zeigen darüber hinaus auch eine sehr gute Wirkungsdauer, wie z.B. gegen die Raupen des Baumwollkapselwurms (Heliothis virescens) oder die Raupen des Heerwurms (Spodoptera frugiperda).

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Emtegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

### Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitem oder Resistenzentwicklungen vorzubeugen. In vielen Fällen erhält man dabei synergistische Effekte, d.h. die Wirksamkeit der Mischung ist größer als die Wirksamkeit der Einzelkomponenten.

### Als Mischpartner kommen zum Beispiel folgende Verbindungen in Frage:

### Fungizide:

Aldimorph, Ampropylfos, Ampropylfos-Kalium, Aridoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Carpropamid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenhexamid, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Iprovalicarb, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
Quinconazol, Quintozen (PCNB), Quinoxyfen,
Schwefel und Schwefel-Zubereitungen, Spiroxamine,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol, Validamycin A, Vinclozolin, Viniconazol,
Zarilamid, Zineb, Ziram sowie
Dagger G, OK-8705, OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,6-Dichlor-5-(methylthio)-4-pyrimidinyl-thiocyanat,
2,6-Dichlor-N-(4-trifluorinethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxarnid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorpheriyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-Hydroxychinolinsulfat,
9H-Xanthen-9-carbonsäure-2-[(phenylamino)-carbonyl]-liydrazid,
bis-(1-Methylethyl)-3-methyl-4-[(3-methylbenzoyl)-oxy]-2,5-thiophendicarboxylat,
eis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,
4-[3,4-Dimethoxyphenyl)-3-(4-fluorphenyl)-acryloyl]-morpholin

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin, Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon,
Dichlorvos, Dicofol, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene, Imidacloprid, Indoxacarb, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Spirodiclofen, Sulfotep, Sulprofos, Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat 1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol 2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzarnid 3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat
N-Cyanomethyl-4-trifluormethyl-nicotinamid
3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise emiedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Emteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®}(Baumwolle), Nucotn^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.
Aus der Ordnung der Mallophagida und den Unterordnungen Amblycerina sowie Ischnocerina z.B. Trimenopon spp., Menopon spp., Trinoton spp., Bovicola spp., Werneckiella-spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp.
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören. Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/öder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyfenozid und Triflumuron,

sowie Fungizide wie Epoxyconazole, Hexaconazole, Azaconazöle, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-*n*-butylzinnchlorid, Ihupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

### Algizide wie

2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

### Fungizide wie

Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;

### Molluskizide wie

Fentinacetat, Metaldehyd, Methiocarb, Niclosamid, Thiodicarb und Trimethacarb;
oder herkömmliche Antifouling-Wirkstoffe wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinirnid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten des weiteren die üblichen Bestandteile wie z.B. in Ungerer, *Chem. Ind.* **1985,** *37,* 730-732 und Williams, Antifouling Marine Coatings, Noyes, Park Ridge, **1973** beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia spp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestem, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggem, Schäumen, Gelen, Verdampferprodukten mit Verdampferplättchen aus Cellulose oder Kunststoff, Flüssigverdampfern, Gel- und Mernbranverdampfern, propellergetriebenen Verdampfern, energielosen bzw. passiven Verdampfungssystemen, Mottenpapieren, Mottensäckchen und Mottengelen, als Granulate oder Stäube, in Streuködem oder Köderstationen.

Die Herstellung und die Verwendung der erfindungsgemäßen Stoffe geht aus den folgenden Beispielen hervor.

### Herstellungsbeispiele

### Beispiel 1

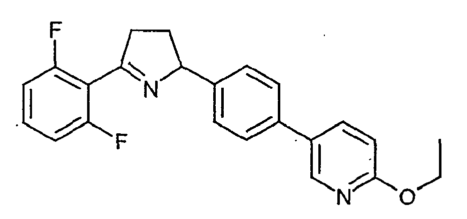

5-(2,6-Difluorphenyl)-2-[4-(4,4,5,5-tetramethyl-1,3,2-dioxaborolan-2-yl)phenyl]-3,4-dihydro-2H-pyrrol (0,96 g, 2,50 mmol) wird in 1,2-Dimethoxyethan (70 ml) unter Argonatmosphäre vorgelegt. Nacheinander gibt man 5-Brom-2-ethoxypyridin (III-1) (0,61 g, 3,00 mmol), 1,1'-Bis(diphenylphosphino)ferrocenpalladium(II)chlorid (0,05 g, 0,07 mol) und 3,75 ml Natriumcarbonat-Lösung (20%ig, w/v) zu. Man lässt das Reaktionsgemisch 16 h bei 80°C nachreagieren.

Das Reaktionsgemisch wird anschließend mit Wasser/ Essigsäureethylester versetzt, die organische Phase abgetrennt, über Natriumsulfat getrocknet, filtriert, mit 5 g Florisil versetzt und eingeengt. Das Rohprodukt wird mittels Chromatographie an Kieselgel (Laufmittel: n-Hexan/Essigsäureethylester 4:1) aufgereinigt.

Man erhält 0,68 g (67% d. Th.) an 5-{4-[5-(2,6-difluorophenyl)-3,4-dihydro-2H-pyrrol-2-yl]phenyl}-2-ethoxypyridine.
- HPLC:: LogP (pH 2,3) = 2,92
- NMR (CD₃CN):: δ = 1.3-1.4 (t, 3H), 1.8-1.9 (m, 1H), 2.7 (m, 1H), 3.0-3.1 (m, 2H), 4.3-4.4 (m, 2H), 5.3 (m, 1H), 6.8 (d, 1H), 7.0-7.1 (t, 2H), 7.3-7.4 (m, 3H), 7.4 (d, 2H), 7.9 (m, 1H), 8.4 (d, 1H) ppm.

Analog Beispiel 1 sowie entsprechend den allgemeinen Beschreibungen der Verfahren (A), (B), (C) oder (D) können die in der folgenden Tabelle genannten Verbindungen hergestellt werden.

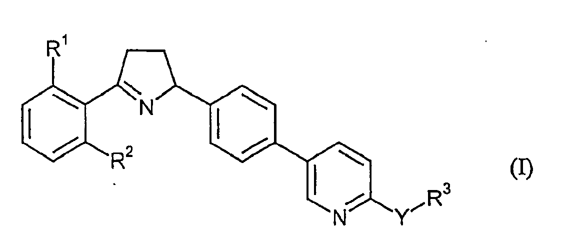

| **Nr.** | **R**^{**1**} | **R**^{**2**} | **Y** | **R**^{**3**} | **Log P** |
|---|---|---|---|---|---|
| 2 | F | F | O | Methyl | |
| 3 | F | F | S | Methyl | |
| 4 | F | F | S | Ethyl | 3,29^{a)}, 4,54^{b)} |
| 5 | F | F | O | n-Propyl | 3,42^{a)}, 4,77^{b)} |
| 6 | F | F | S | n-Propyl | |
| 7 | F | F | O | i-Propyl | 3,42^{a)}, 4,73^{b)} |
| 8 | F | F | S | i-Propyl | 3,80^{a)}, 4,96^{b)} |
| 9 | F | F | O | n-Butyl | |
| 10 | F | F | S | n-Butyl | |
| 11 | F | F | O | i-Butyl | |
| 12 | F | F | S | i-Butyl | |
| 13 | F | F | O | s-Butyl | |
| 14 | F | F | S | s-Butyl | |
| 15 | F | F | O | t-Butyl | 3,94^{a)} |
| 16 | F | F | S | t-Butyl | |
| 17 | F | F | O | Cyclopropyl | |
| 18 | F | F | S | Cyclopropyl | |
| 19 | F | F | O | Cyclobutyl | 3,66^{a}), 4,90^{b}) |
| 20 | F | F | S | Cyclobutyl | |
| 21 | F | F | O | Cyclopentyl | 3,99^{a)}, 5,34^{b)} |
| 22 | F | F | S | Cyclopentyl | |
| 23 | F | F | O | Cyclohexyl | |
| 24 | F | F | S | Cyclohexyl | |
| 25 | F | F | O | Cyclopropylmethyl | 3,48^{a)}, 4,67^{b)} |
| 26 | F | F | S | Cyclopropylmethyl | |
| 27 | F | F | O | Cyclobutylmethyl | |
| 28 | F | F | S | Cyclobutylmethyl | |
| 29 | F | F | O | Cyclopentylmethyl | |
| 30 | F | F | S | Cyclopentylmethyl | |
| 31 | F | F | O | Cyclohexylmethyl | |
| 32 | F | F | S | Cyclohexylmethyl | |
| 33 | F | F | O | Cyclopropylethyl | |
| 34 | F | F | S | Cyclopropylethyl | |
| 35 | F | F | O | Cyclobutylethyl | |
| 36 | F | F | S | Cyclobutylethyl | |
| 37 | F | F | O | Cyclopentylethyl | |
| 38 | F | F | S | Cyclopentylethyl | |
| 39 | F | F | O | Cyclohexylethyl | |
| 40 | F | F | S | Cyclohexylethyl | |

### Herstellung von Ausgangsstoffen der Formel (III)

### Beispiel III-1

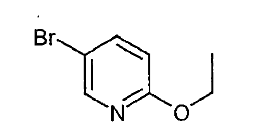

0,49 g (20,26 mmol) Natriumhydrid werden unter Argon vorgelegt und auf 10°C abgekühlt. Man gibt 10 ml Dimethylformamid zu. Anschließend tropft man eine Lösung von 0,93 g (20,26 mmol) Ethanol in 40 ml Dimethylformamid langsam zu und lässt noch 30 min nachreagieren. Anschließend tropft man 4,00 g (16,89 mmol) 2,5-Dibrompyridin zu und lässt für weitere 16 h nachreagieren.
Das Reaktionsgemisch wird mit Wasser versetzt. Man extrahiert dreimal mit Essigsäureethylester. Die organische Phase wird je einmal mit NatriumhydrogencarbonatLösung und Natriumchlorid-Lösung gewaschen, über Natriumsulfat getrocknet, filtriert und eingeengt.

Man erhält 2,35 g (67% d. Th.) an 5-Brom-2-ethoxypyridin.
- HPLC:: LogP (pH 2,3) = 2,92
- NMR(CD₃CN):: δ = 1.33 (t, 3H), 4.27-4.32 (m, 2H), 6.68 (d, 1H), 7.75 (m, 1H), 8.2 (d, 1H) ppm.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im saüren Bereich bei pH 2.3 mit 0,1% wässriger Phosphorsäure und Acetonitril als Eluenten; linearer Gradient von 10% Acetonitril bis 90% Acetonitril. Die Werte sind in den Tabellen mit ^{a)} markiert.

Die Bestimmung erfolgt im neutralen Bereich bei pH 7.5 mit 0,01-molare wässriger Phosphatpuffer-Lösung und Acetonitril als Eluenten; linearer Gradient von 10 % Acetonitril bis 90 % Acetonitril. Die Werte sind in den Tabellen mit ^{b)} markiert.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Aphis gossypii-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter (Gossypium hirsutum), die stark von der Baumwollblattlaus (Aphis gossypii) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele gute Wirksamkeit:

**Tabelle A**

| pflanzenschädigende Insekten | | | |
|---|---|---|---|
| **Aphis gossypii-Test** | | | |
| Nr. | Wirkstoffe | Wirkstoffkonzentration in ppm | Wirkungsgrad in % nach 6^{d} |
| 5 | 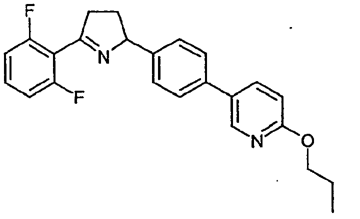 | 100 | 99 |
| 7 | 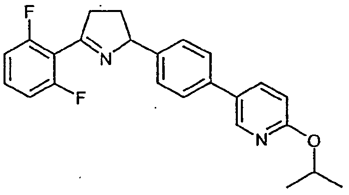 | 100 | 99 |

### Beispiel B

### Heliothis armigera-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojatriebe (Glycine max) werden durch Tauchen in die Wirkstoffzubereitung der gewünschten Konzentration behandelt und mit Raupen des Baumwollkapselwurms (Heliothis armigera) besetzt, solange die Blätter noch feucht sind.
Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z.B. die folgenden Verbindungen der Herstellungsbeispiele gute Wirksamkeit:

**Tabelle B**

| pflanzenschädigende Insekten | | | |
|---|---|---|---|
| **Heliothis-Larven-Test** | | | |
| Nr. | Wirkstoffe | Wirkstoffkonzentration in ppm | Wirkungsgrad in % nach 7^{d} |
| 15 | 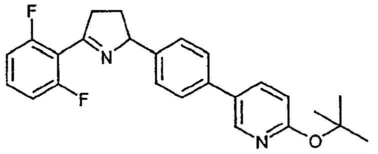 | 20 | 100 |
| 25 | 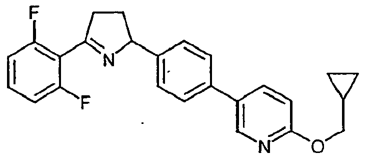 | 20 | 100 |

### Beispiel C

### Heliothis virescens-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojatriebe (Glycine max) werden durch Tauchen in die Wirkstoffzubereitung der gewünschten Konzentration behandelt und mit Heliothis virescens-Raupen besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit:

**Tabelle C**

| pflanzenschädigende Insekten | | | |
|---|---|---|---|
| **Heliothis virescens-Test** | | | |
| Nr. | Wirkstoffe | Wirkstoffkonzentration in ppm | Wirkungsgrad in % nach 7^{d} |
| 5 | 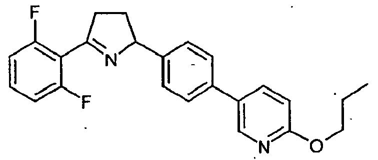 | 100 | 100 |
| 4 | 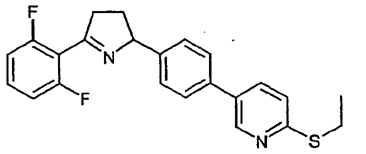 | 100 | 100 |
| 8 | 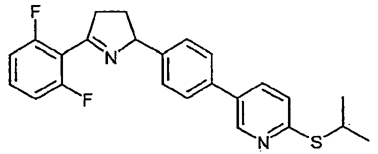 | 100 | 100 |
| 7 | 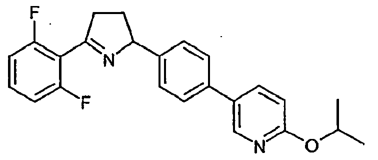 | 100 | 100 |

### Beispiel D

### Phaedon-Larven-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele überlegene Wirksamkeit gegenüber dem Stand der Technik:

**Tabelle D**

| pflanzenschädigende Insekten | | | |
|---|---|---|---|
| **Phaedon-Larven-Test** | | | |
| Nr. | Wirkstoffe | Wirkstoffkonzentration in ppm | Wirkungsgrad in % nach 7^{d} |
| 1 | 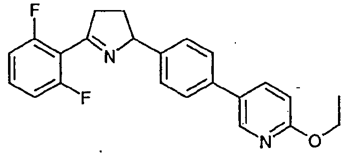 | 100 | 100 |
| 5 | 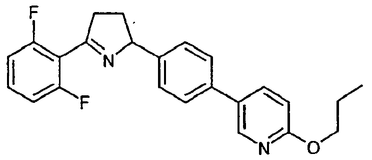 | 100 | 100 |
| 4 | 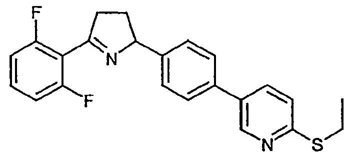 | 100 | 100 |
| 8 | 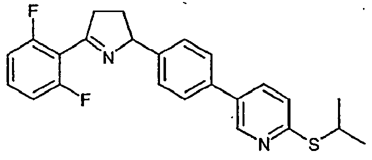 | 100 | 100 |
| 7 | 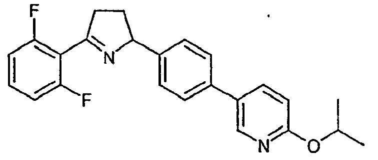 | 100 | 100 |
| 15 | 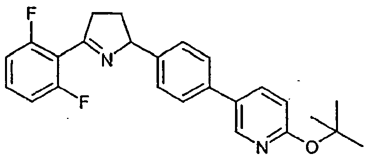 | 100 | 100 |
| 21 | 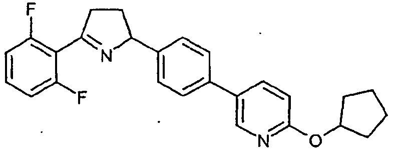 | 100 | 100 |
| 25 | 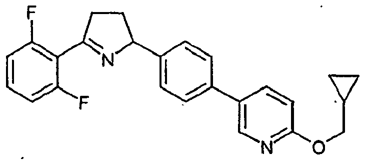 | 100 | 100 |
| 19 | 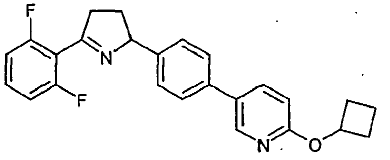 | 20 | 100 |

### Beispiel E

### Plutella-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt z. B. die folgende Verbindung der Herstellungsbeispiele gute Wirksamkeit:

**Tabelle E**

| pflanzenschädigende Insekten | | | |
|---|---|---|---|
| **Plutella-Test** | | | |
| Nr. | Wirkstoffe | Wirkstoffkonzentration in ppm | Wirkungsgrad in % nach 7^{d} |
| 5 | 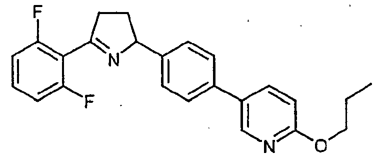 | 100 | 100 |
| 4 | 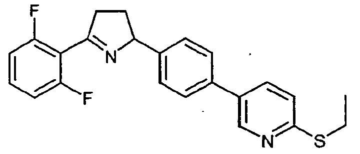 | 100 | 100 |
| 8 | 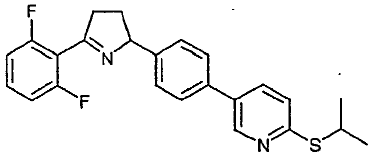 | 100 | 100 |
| 7 | 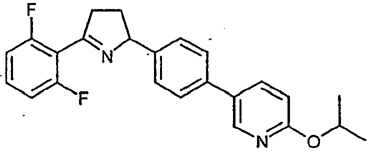 | 100 | 100 |
| 15 | 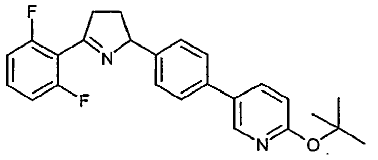 | 20. | 100 |
| 25 | 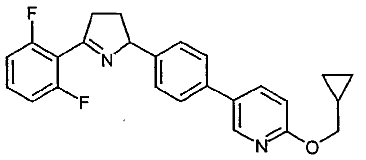 | 20 | 100 |

### Beispiel F

### Spodoptera exigua-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera exigua) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z.B. die folgenden Verbindungen der Herstellungsbeispiele gute Wirksamkeit:

**Tabelle F**

| pflanzenschädigende Insekten | | | |
|---|---|---|---|
| **Spodoptera exigua-Larven -Test** | | | |
| Nr. | Wirkstoffe | Wirkstoffkonzentration in ppm | Wirkungsgrad in % nach 7^{d} |
| 5 | 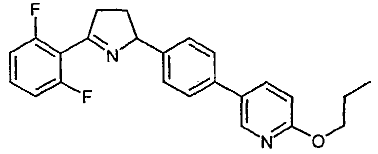 | 100 | 100 |
| 4 | 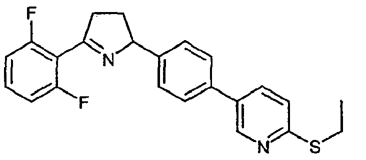 | 100 | 100 |
| 8 | 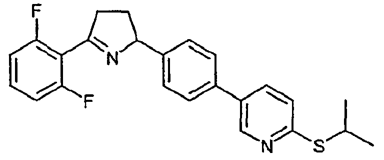 | 100 | 100 |
| 7 | 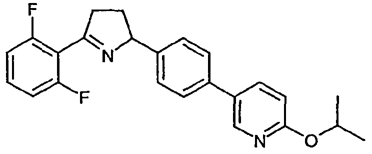 | 100 | 100 |
| 15 | 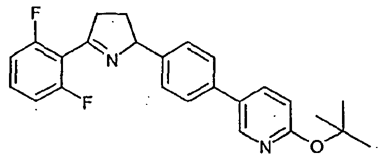 | 20 | 100 |
| 25 | 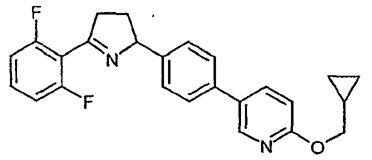 | 20 | 100 |

### Beispiel G

### Spodoptera frugiperda-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit:

**Tabelle G**

| pflanzenschädigende Insekten | | | |
|---|---|---|---|
| **Spodoptera frugiperda -Test** | | | |
| Nr. | Wirkstoffe | Wirkstoffkonzentration in ppm | Wirkungsgrad in % nach 7^{d} |
| 1 | 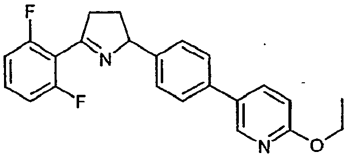 | 100 | 100 |
| 5 | 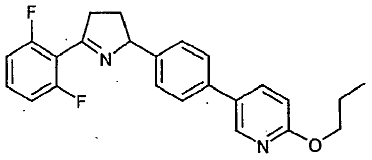 | 100 | 100 |
| 4 | 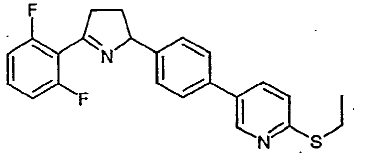 | 100 | 100 |
| 8 | 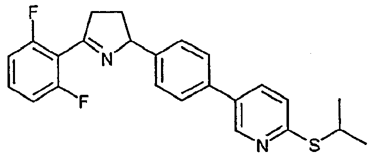 | 100 | 100 |
| 7 | 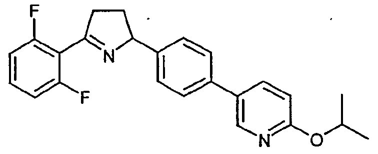 | 100 | 100 |
| 15 | 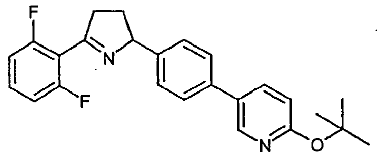 | 100 | 100 |
| 21 | 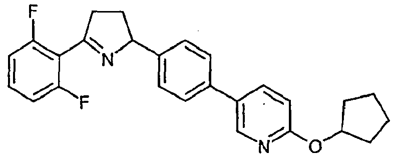 | 100 | 100 |
| 25 | 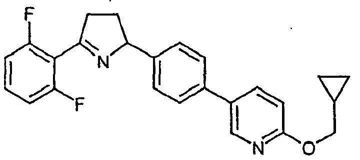 | 100 | 100 |
| 19 | 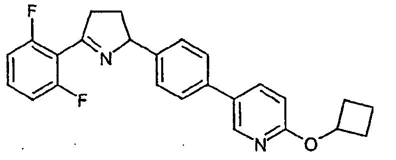 | 100 | 100 |

### Beispiel H

### Tetranychus-Test (OP-resistent/Tauchbehandlung)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der Gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele gute Wirksamkeit:

**Tabelle H**

| pflanzenschädigende Milben | | | |
|---|---|---|---|
| **Tetranychus-Test** (OP-resistent/Tauchbehandlung) | | | |
| Nr. | Wirkstoffe | Wirkstoffkonzentration in ppm | Wirkungsgrad in % nach 7^{d} |
| 5 | 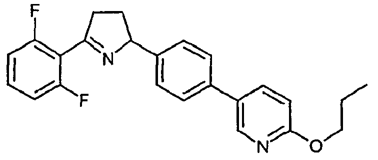 | 100 | 98 |
| 4 | 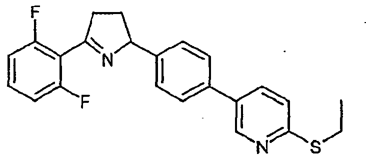 | 100 | 95 |
| 8 | 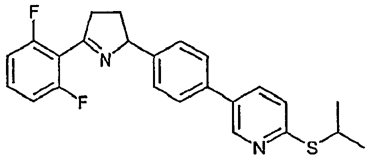 | 100 | 98 |
| 21 | 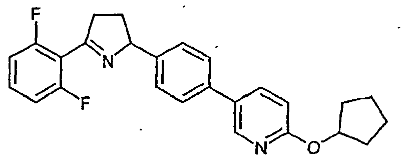 | 100 | 95 |
| 15 | 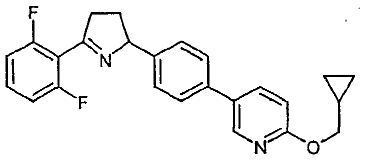 | 100 | 95 |
| 19 | 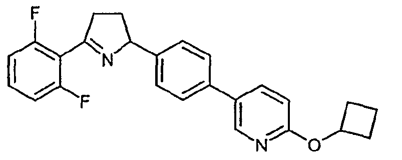 | 100 | 95 |

### Beispiel I

### Panonychus-Test

| | | |
|---|---|---|
| Lösungsmittel: | 3 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Ca. 30 cm hohe Pflaumenbäumchen (Prunus domestica), die stark von allen Stadien der Obstbaumspinnmilbe (*Panonychus ulmi*) befallen sind, werden mit einer Wirkstoffzube-reitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z. B. die folgende Verbindung der Herstellungsbeispiele gute Wirksamkeit:

**Tabelle I**

| pflanzenschädigende Milben | | | |
|---|---|---|---|
| **Panonychus-Test** | | | |
| Nr. | Wirkstoffe | Wirkstoffkonzentration in ppm | Wirkungsgrad in % nach 7^{d} |
| 7 | 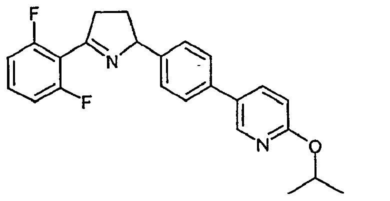 | 100 | 100 |

### Beispiel K

### Diabrotica balteata - Test (Larven im Boden)

Grenzkonzentrations-Test / Bodeninsekten - Behandlung transgener Pflanzen

| | | |
|---|---|---|
| Lösungsmittel: | 7 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20% Wirkung).

### Beispiel L

**Heliothis virescens - Test** (Behandlung transgener Pflanzen)

| | | |
|---|---|---|
| Lösungsmittel: | 7 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

### Beispiel M

### Blowfly-Larven-Test / Entwicklungshemmende Wirkung

| | |
|---|---|
| Testtiere: | *Lucilia cuprina*-Larven |
| Lösungsmittel: | Dimethylsulfoxid |

20 mg Wirkstoff werden in 1 ml Dimethylsulfoxid gelöst, geringere Konzentrationen werden durch Verdünnen mit destilliertem Wasser hergestellt.

Etwa 20 Lucilia cuprina-Larven werden in ein Teströhrchen gebracht, welches ca. 1 cm³ Pferdefleisch und 0.5 ml der zu testende Wirkstoffzubereitung enthält. Nach 24 und 48 Stunden wird die Wirksamkeit der Wirkstoffzubereitung ermittelt. Die Teströhrchen werden in Becher mit Sand-bedecktem Boden überführt. Nach weiteren 14 Tagen werden die Teströhrchen entfernt und die Puppen Fliegen ausgezählt.

Die Wirkung der Wirkstoffzubereitung wird nach der Zahl der geschlüpften Fliegen nach 1.5-facher Entwicklungsdauer einer unbehandelten Kontrolle beurteilt. Dabei bedeutet 100 %, dass keine Fliegen geschlüpft sind; 0 % bedeutet, dass alle Fliegen normal geschlüpft sind.

Bei diesem Test zeigen z. B. die folgenden Verbindungen der Herstellungsbeispiele gute Wirksamkeit:

**Tabelle M**

| **Blowfly-Larven-Test / Entwicklungshemmende Wirkung** | | | |
|---|---|---|---|
| Nr. | Wirkstoffe | Wirkstoffkonzentration in ppm | % Wirkung/ Abtötung (48 h) |
| 25 | 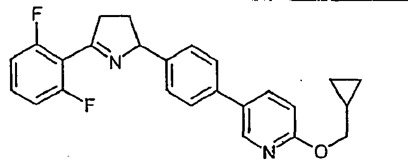 | 100 | 100 |
| | | | |

| Nr. | Wirkstoffe | Wirkstoffkonzentration in ppm | % Wirkung/ Abtötung (14 d) |
|---|---|---|---|
| 25 | 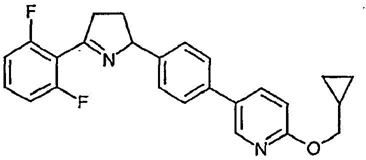 | 20 | 100 |
| 19 | 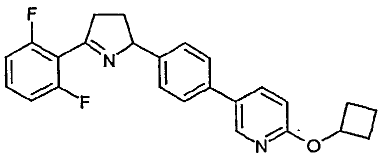 | 100/20 | 100/100 |
| 4 | 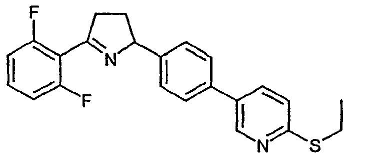 | 100 | 100 |

## Patentansprüche

1. Δ¹-Pyrroline der Formel (I) 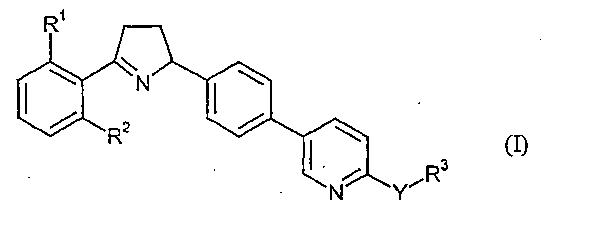 in welcher
R¹ für Halogen oder Methyl steht,
R² für Wasserstoff oder Halogen steht,
Y für O (Sauerstoff) oder S (Schwefel) steht,
R³ für C₁-C₄-Alkyl, C₃-C₆-Cycloalkyl oder C₃-C₆-Cycloalkyl-C₁-C₂₋alkyl steht.

2. Δ¹-Pyrroline der Formel (I) gemäß Anspruch 1, in welcher
R¹ für Fluor, Chlor oder Methyl steht,
R² für Wasserstoff, Fluor oder Chlor steht,
Y für O (Sauerstoff) oder S (Schwefel) steht,
R³ für C₁-C₄-Alkyl, C₃-C₆-Cycloalkyl oder C₃-C₆-Cycloalkyl-C₁-C₂₋alkyl steht.

3. Δ¹-Pyrroline der Formel (I) gemäß Anspruch 1, in welcher
R¹ für Fluor oder Chlor steht,
R² für Wasserstoff, Fluor oder Chlor steht,
Y für O (Sauerstoff) oder S (Schwefel) steht,
R³ für Methyl, Ethyl, Propyl, Butyl, Cyclopropyl, Cyclobutyl, Cyclopentyl, Cyclohexyl, Cyclopropyl-C₁-C₂-alkyl, Cyclobutyl-C₁-C₂₋alkyl, Cyclopentyl-C₁-C₂-alkyl oder Cyclohexyl-C₁-C₂-alkyl stehen.

4. Δ¹-Pyrroline der Formel (I) gemäß Anspruch 1, in welcher
R¹ für Fluor oder Chlor steht,
R² für Wasserstoff oder Fluor steht,
Y für O (Sauerstoff) oder S (Schwefel) steht,
R³ für Methyl, Ethyl, n-Propyl, iso-Propyl, n-Butyl, iso-Butyl, sec-Butyl, tert-Butyl, Cyclopropyl, Cyclobutyl, Cyclopentyl, Cyclohexyl, Cyclopropylmethyl, Cyclobutylmethyl, Cyclopentylmethyl, Cyclohexylmethyl, Cyclopropylethyl, Cyclobutylethyl, Cyclopentylethyl oder Cyclohexylethyl stehen.

5. Δ¹-Pyrroline der Formel (I) gemäß Anspruch 1, in welcher R¹ und R² für Fluor stehen.

6. Δ¹-Pyrroline der Formel (I) gemäß Anspruch 1, in welcher Y für Sauerstoff steht.

7. Δ¹-Pyrroline der Formel (I) gemäß Anspruch 1, in welcher Y für Schwefel steht.

8. Δ¹-Pyrroline der Formel (I) gemäß Anspruch 1, in welcher R³ für C₁-C₄₋Alkyl steht.

9. (R)-konfigurierte Verbindungen der Formel (I-a) 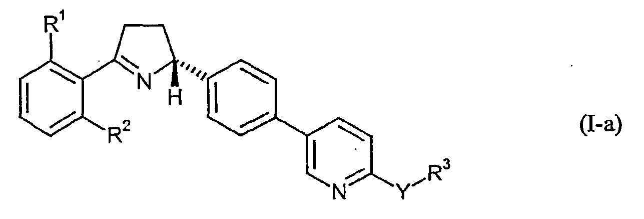 in welcher
R¹, R², Y und R³ die in einem oder mehreren der Ansprüche 1 bis 8 angegebenen Bedeutungen haben.

10. Verfahren zur Herstellung von Verbindungen der Formel (I) gemäß Anspruch 1, **dadurch gekennzeichnet, dass** man
A) Δ¹-Pyrroline der Formel (II) 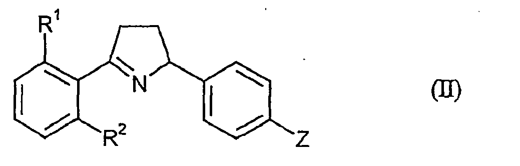 in welcher
R¹ und R² die in Anspruch 1 angegebenen Bedeutungen haben,
Z für Chlor, Brom, Iod, -OSO₂CF₃ oder -OSO₂(CF₂)₃CF₃ steht,
mit Heterocyclen der Formel (III) 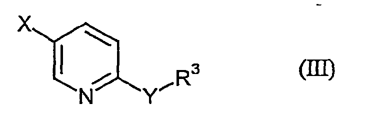 in welcher
Y und R³ die in Anspruch 1 angegebenen Bedeutungen haben, und
X für Chlor, Brom, Iod, -OSO₂CF₃ oder -OSO₂(CF₂)₃CF₃ steht,
in Gegenwart eines Katalysators, in Gegenwart eines Diboronsäureesters und gegebenenfalls in Gegenwart eines Säurebindemittels und gegebenenfalls in Gegenwart eines Verdünnungsmittels in einer Tandem-Reaktion umsetzt,
oder
B) Δ¹-Pyrroline der Formel (IV) 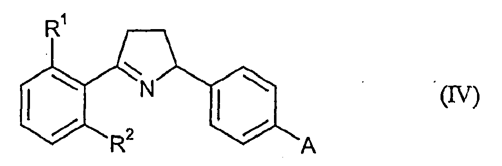 in welcher
R¹ und R² die in Anspruch 1 angegebenen Bedeutungen haben, und
A für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl steht,
mit Heterocyclen der Formel (III) 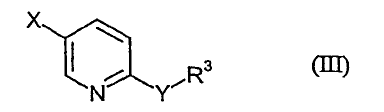 in welcher
Y und R³ die in Anspruch 1 angegebenen Bedeutungen haben,
X die oben angegebenen Bedeutungen hat,
in Gegenwart eines Katalysators, gegebenenfalls in Gegenwart eines Säurebindemittels und gegebenenfalls in Gegenwart eines Verdünnungsmittels umsetzt,
oder
C) Δ¹-Pyrroline der Formel (II) 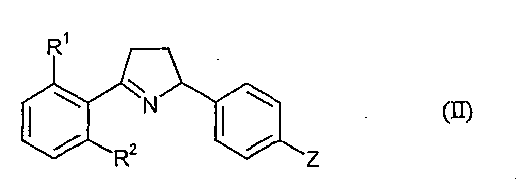 in welcher
R¹ und R² die in Anspruch 1 angegebenen Bedeutungen haben
und Z die oben angegebenen Bedeutungen hat,
mit Boronsäure-Derivaten der Formel (V) 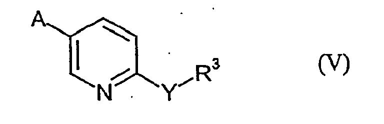 in welcher
Y und R³ die in Anspruch 1 angegebenen Bedeutungen haben,
A die oben angegebenen Bedeutungen hat,
in Gegenwart eines Katalysators, gegebenenfalls in Gegenwart eines Säurebindemittels und gegebenenfalls in Gegenwart eines Verdünnungsmittels umsetzt,
oder
D) Δ¹-Pyrroline der Formel (II-a) 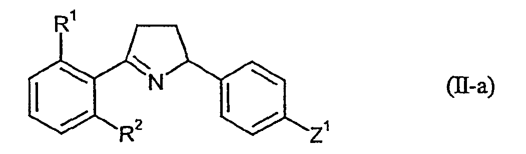
in welcher
R¹ und R² die in Anspruch 1 angegebenen Bedeutungen haben,
Z¹ für Brom oder Iod steht,
mit organometallischen Verbindungen der Formel (VI) 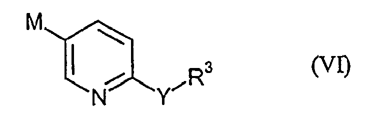 in welcher
Y und R³ die in Anspruch 1 angegebenen Bedeutungen haben,
M für ZnCl, Sn(Me)₃ oder Sn(n-Bu)₃ steht,
in Gegenwart eines Katalysators, gegebenenfalls in Gegenwart eines Säurebindemittels und gegebenenfalls in Gegenwart eines Verdünnungsmittels umsetzt.

11. Schädlingsbekämpfungsmittel, **gekennzeichnet durch** einen Gehalt an mindestens einer Verbindung der Formel (I) gemäß Anspruch 1 neben Streckmitteln und/oder oberflächenaktiven Stoffen.

12. Verwendung von Verbindungen der Formel (I) gemäß Anspruch 1 zur Bekämpfung von Schädlingen.

13. Verfahren zur Bekämpfung von Schädlingen, **dadurch gekennzeichnet, dass** man Verbindungen der Formel (I) gemäß Anspruch 1 aupf Schädlinge und/oder ihren Lebensraum einwirken lässt.

14. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Stoffen vermischt.

## Claims

1. Δ¹-Pyrrolines of formula (I) 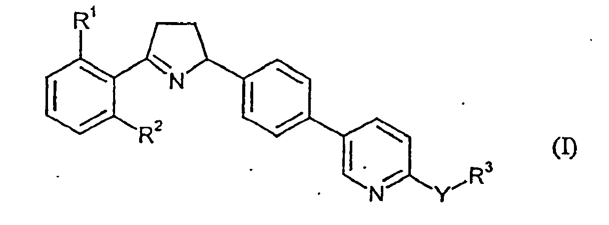 in which
R¹ represents halogen or methyl,
R² represents hydrogen or halogen,
Y represents O (oxygen) or S (sulfur),
R³ represents C₁-C₄-alkyl, C₃-C₆-cycloalkyl or C₃-C₆₋cycloalkyl-C₁-C₂-alkyl.

2. Δ¹-Pyrrolines of formula (I) according to claim 1, in which
R¹ represents fluorine, chlorine or methyl,
R² represents hydrogen, fluorine or chlorine,
Y represents O (oxygen) or S (sulfur),
R³ represents C₁-C₄-alkyl, C₃-C₆-cycloalkyl or C₃-C₆₋cycloalkyl-C₁-C₂-alkyl.

3. Δ¹-Pyrrolines of formula (I) according to claim 1, in which
R¹ represents fluorine or chlorine,
R² represents hydrogen, fluorine or chlorine,
Y represents O (oxygen) or S (sulfur),
R³ represents methyl, ethyl, propyl, butyl, cyclopropyl, cyclobutyl, cyclopentyl, cyclohexyl, cyclopropyl-C₁-C₂-alkyl, cyclobutyl-C₁-C₂-alkyl, cyclopentyl-C₁-C₂-alkyl or cyclohexyl-C₁-C₂-alkyl.

4. Δ¹-Pyrrolines of formula (I) according to claim 1, in which
R¹ represents fluorine or chlorine,
R² represents hydrogen or fluorine,
Y represents O (oxygen) or S (sulfur),
R³ represents methyl, ethyl, n-propyl, isopropyl, n-butyl, isobutyl, sec.-butyl, tert.-butyl, cyclopropyl, cyclobutyl, cyclopentyl, cyclohexyl, cyclopropylmethyl,cyclobutylmethyl, cyclopentylmethyl, cyclohexylmethyl, cyclopropylethyl, cyclobutylethyl, cyclopentylethyl or cyclohexylethyl.

5. Δ¹-Pyrrolines of formula (I) according to claim 1, in which R¹ and R² represent fluorine.

6. Δ¹-Pyrrolines of formula (I) according to claim 1, in which Y represents oxygen.

7. Δ¹-Pyrrolines of formula (I) according to claim 1, in which Y represents sulfur.

8. Δ¹-Pyrrolines of formula (I) according to claim 1, in which R³ represents C₁-C₄-alkyl.

9. Compounds having the (R) configuration of formula (I-a) 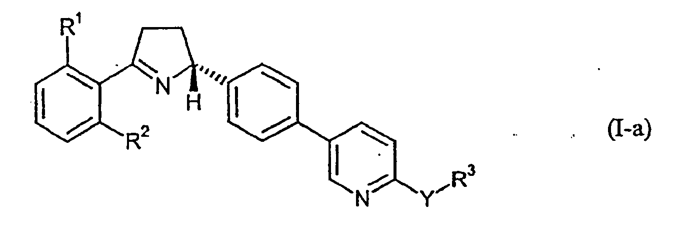 in which
R¹, R², Y and R³ are as defined in one or more of claims 1 to 8.

10. Process for the preparation of compounds of formula (I) according to claim 1, **characterised in that**
A) Δ¹-pyrrolines of formula (II) 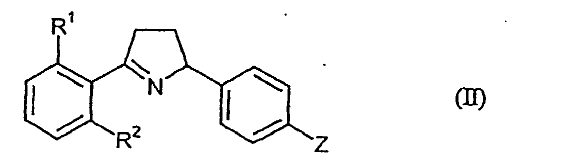 in which
R¹ and R² are as defined in claim 1,
Z represents chlorine, bromine, iodine, -OSO₂CF₃ or -OSO₂(CF₂)₃CF₃,
are reacted in a tandem reaction in the presence of a catalyst, in the presence of a diboronic acid ester and optionally in the presence of an acid-binding agent and optionally in the presence of a diluent, with heterocycles of formula (III) 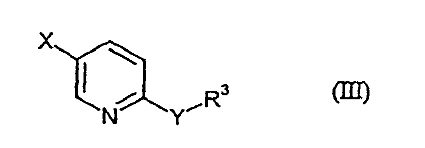 in which
Y and R³ are as defined in claim 1 and
X represents chlorine, bromine, iodine, -OSO₂CF₃ or -OSO₂(CF₂)₃CF₃,
or
B) Δ¹-pyrrolines of formula (IV) 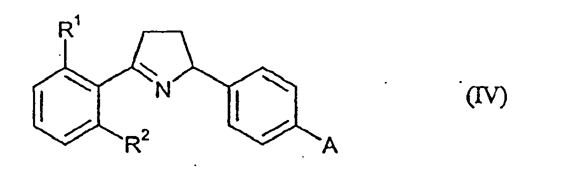 in which
R¹ and R² are as defined in claim 1 and
A represents -B(OH)₂, (4,4,5,5-tetramethyl-1,3,2-dioxaborolan)-2-yl, (5,5-dimethyl-1,3,2-dioxaborinan)-2-yl, (4,4,6-trimethyl-1,3,2-dioxaborinan)-2-yl or 1,3,2-benzodioxaborol-2-yl,
are reacted in the presence of a catalyst, optionally in the presence of an acid-binding agent and optionally in the presence of a diluent, with heterocycles of formula (III) 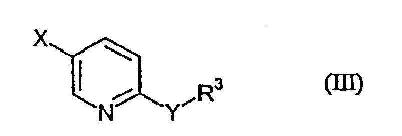 in which
Y and R ³ are as defined in claim 1,
X is as defined hereinbefore,
or
C) Δ¹-pyrrolines of formula (II) 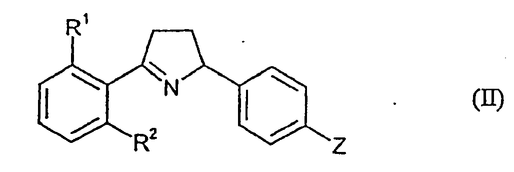 in which
R¹ and R² are as defined in claim 1
and Z is as defined hereinbefore,
are reacted in the presence of a catalyst, optionally in the presence of an acid-binding agent and optionally in the presence of a diluent, with boronic acid derivatives of formula (V) 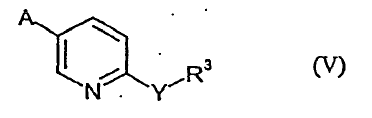 in which
Y and R ³ are as defined in claim 1,
A is as defined hereinbefore,
or
D) Δ¹-pyrrolines of formula (II-a) 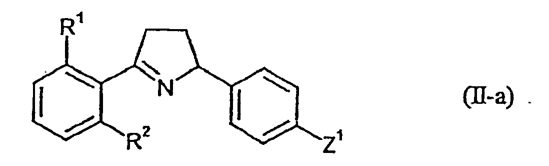 in which
R¹ and R² are as defined in claim 1,
Z¹ represents bromine or iodine,
are reacted in the presence of a catalyst, optionally in the presence of an acid-binding agent and optionally in the presence of a diluent, with organometallic compounds of formula (VI) 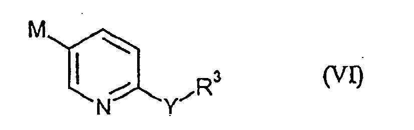 in which
Y and R³ are as defined in claim 1,
M represents ZnCl, Sn(Me)₃ or Sn(n-Bu)₃.

11. Pesticide, **characterised by** a content of at least one compound of formula (I) according to claim 1 together with extenders and/or surface-active substances.

12. Use of compounds of formula (I) according to claim 1 in the control of pests.

13. Method of controlling pests, **characterised in that** compounds of formula (I) according to claim 1 are allowed to act on pests and/or the locus thereof.

14. Process for the preparation of pesticides, **characterised in that** compounds of formula (I) according to claim 1 are mixed with extenders and/or surface-active agents.

## Revendications

1. Δ¹-pyrrolines de la formule (I) 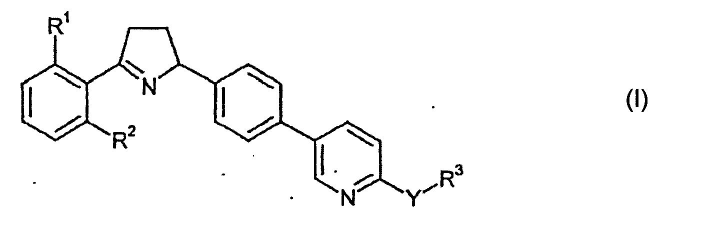 dans laquelle
R¹ représente un halogène ou un méthyle,
R² correspond à l'hydrogène ou à un halogène,
Y représente O (oxygène) ou S (soufre),
R³ est un alkyle en C₁-C₄, un cycloalkyle en C₃-C₆ ou un cycloalkyle en C₃-C₆-alkyle en C₁-C₂.

2. Δ¹-pyrrolines de la formule (I) selon la revendication 1, dans laquelle
R¹ représente le fluor, le chlore ou un méthyle,
R² correspond à l'hydrogène, au fluor ou au chlore,
Y représente O (oxygène) ou S (soufre),
R³ est un alkyle en C₁-C₄, un cycloalkyle en C₃-C₆ ou un cyc!oa!ky!e en C₃-C₆-alkyle en C₁-C₂.

3. Δ¹-pyrrolines de la formule (I) selon la revendication 1, dans laquelle
R¹ représente le fluor ou le chlore,
R² correspond à l'hydrogène, au fluor ou au chlore,
Y représente O (oxygène) ou S (soufre),
R³ est un méthyle, un éthyle, un propyle, un butyle, un cyclopropyle, un cyclobutyle, un cyclopentyle, un cyclohexyle, un cyclopropyl-alkyle en C₁-C₂. un cyclobutyle-alkyle en C₁-C₂, un cyclopentyle-alkyle en C₁-C₂ ou un cyclohexylealkyle en C₁-C₂.

4. Δ¹-pyrrolines de la formule (I) selon la revendication 1, dans laquelle
R¹ représente le fluor ou le chlore,
R² correspond à l'hydrogène ou au fluor,
Y représente O (oxygène) ou S (soufre),
R³ est un méthyle, un éthyle, un n-propyle, un iso-propyle, un n-butyle, un iso-butyle, un sec-butyle, un tert-butyle, un cyclopropyle, un cyclobutyle, un cyclopentyle, un cyclohexyle, un cyclopropylméthyle, un cyclobutylméthyle, un cyclopentylméthyle, un cyclohexylméthyle, un cyclopropyléthyle, un cyclobutyléthyle, un cyclopentyléthyle ou un cyclohexyléthyle.

5. Δ¹-pyrrolines de la formule (I) selon la revendication 1, dans laquelle R¹ et R² représentent le fluor.

6. Δ¹-pyrrolines de la formule (I) selon la revendication 1, dans laquelle Y représente l'oxygène.

7. Δ¹-pyrrolines de la formule (I) selon la revendication 1, dans laquelle Y représente le soufre.

8. Δ¹-pyrrolines de la formule (I) selon la revendication 1, dans laquelle R³ représente un alkyle en C₁-C₄.

9. Composés (R)-configurés de la formule (I-a) 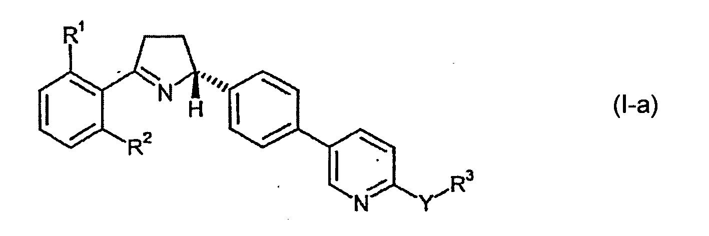 dans laquelle R¹, R²,Y et R³ possèdent la signification mentionnée dans une ou plusieurs des revendications 1 à 8.

10. Procédé de préparation de composés de la formule (I) selon la revendication 1, **caractérisé en ce que** l'on fait réagir
A) des Δ¹-pyrrolines de la formule (II) 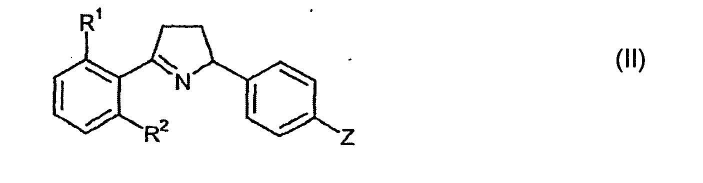 dans laquelle
R¹ et R² possèdent la signification mentionnée dans la revendication 1,
Z représente le chlore, le brome, l'iode, -OSO₂CF₃ ou -OSO₂(CF₂)₃CF₃,
avec des hétérocycles de la formule (III) 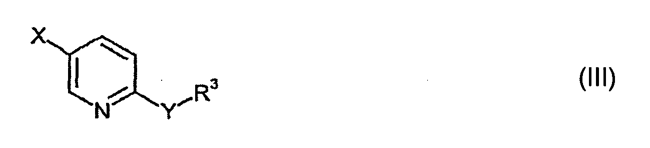 dans laquelle
Y et R³ possèdent la signification mentionnée dans la revendication 1 et
X représente le chlore, le brome, l'iode, -OSO₂CF₃ ou -OSO₂(CF₂)₃CF₃,
en présence d'un catalyseur, en présence d'un ester de l'acide diborique et, le cas échéant, en présence d'un liant acide et le cas échéant, en présence d'un diluant, dans une réaction tandem,
ou
B) des Δ¹-pyrrolines de la formule (IV) 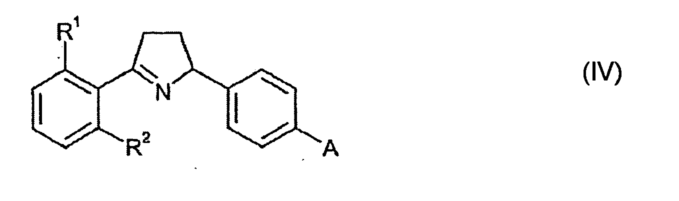 dans laquelle
R¹ et R² possèdent la signification mentionnée dans la revendication 1,
A représente les composés suivants: -B(OH)₂, (4,4,5,5-tétraméthyl-1,3,2-dioxaborolan)-2-yle, (5,5-diméthyl-1,3,2-dioxaborinan)-2-yle, (4,4,6-triméthyl-1,3,2-dioxaborinan)-2-yle ou 1,3,2-benzodioxaborol-2-yle
avec des hétérocycles de la formule (III) 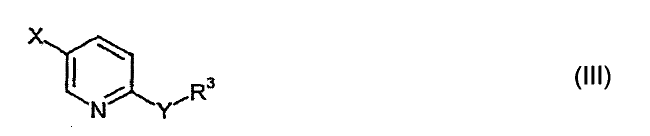 dans laquelle
Y et R³ possèdent la signification mentionnée dans la revendication 1,
X possède la signification mentionnée ci-dessus,
en présence d'un catalyseur, le cas échéant, en présence d'un liant acide et le cas échéant, en présence d'un diluant,
ou
C) des Δ¹-pyrrolines de la formule (II) 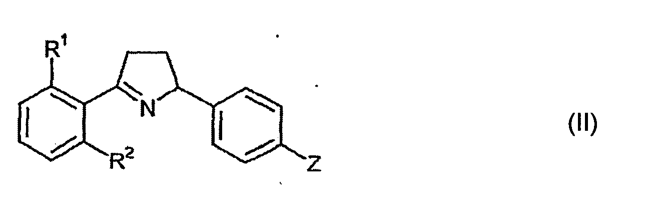 dans laquelle
R¹ et R² possèdent la signification mentionnée dans la revendication 1,
et Z possède la signification mentionnée ci-dessus,
avec des dérivés de l'acide borique de la formule (V) 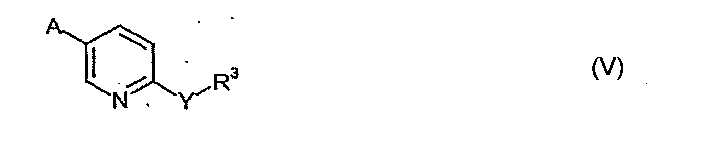 dans laquelle
Y et R³ possèdent la signification mentionnée dans la revendication 1,
A possède la signification mentionnée ci-dessus,
en présence d'un catalyseur, le cas échéant, en présence d'un liant acide et le cas échéant, en présence d'un diluant,
ou
D) des Δ¹-pyrrolines de la formule (II-a) 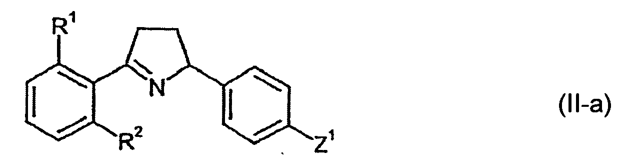 dans laquelle
R¹ et R² possèdent la signification mentionnée dans la revendication 1,
Z¹ représente le brome ou l'iode,
avec des composés organométalliques de la formule (VI) 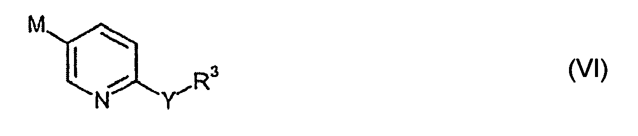 dans laquelle
Y et R³ possèdent la signification mentionnée dans la revendication 1,
M représente les composés suivants: ZnCl, Sn(Me)₃ ou Sn(n-Bu)₃,
en présence d'un catalyseur, le cas échéant, en présence d'un liant acide et le cas échéant, en présence d'un diluant.

11. Parasiticides, **caractérisés en ce qu'**ils contiennent au moins un composé de la formule (I) selon la revendication 1 avec des diluants et/ou des substances tensioactives.

12. Utilisation de composés de la formule (I) selon la revendication 1 pour combattre les parasites.

13. Procédé de lutte contre les parasites, **caractérisé en ce que** l'on fait agir des composés de la formule (I) selon la revendication 1 sur des parasites et/ou sur leur habitat.

14. Procédé de production de parasiticides, **caractérisé en ce que** l'on mélange des composés de la formule (I) selon la revendication 1 avec des diluants et/ou des agents tensioactifs.
